# DEMANDE DE BREVET EUROPEEN

(11) **EP 1 205 210 A1**
(43) Date de publication de la demande: **15.05.2002**
(21) Numéro de dépôt: 01460068.8
(22) Date de dépôt: 09.11.2001
(51) Int. Cl.: A61M 27/00

(54) **Valve sous-cutanée pour le traitement de l'hydrocéphalie et ses dispositifs de réglage**

(30) Priorité: 13.11.2000 FR 0014551
(71) Demandeur: Marion, Bernard, 35120 Mont-Dol (FR)
(72) Inventeur: Marion, Bernard, 35120 Mont-Dol (FR)
(74) Mandataire: Bomer, Françoise Marie

(57) **Abrégé**

L'invention concerne une valve sous-cutanée dont la pression d'ouverture est réglable depuis l'extérieur, cette valve comportant un corps (1) présentant une chambre (21) à paroi intérieure latérale cylindrique (20), un conduit d'amenée (25) et un conduit d'évacuation (26) du liquide céphalo-rachidien débouchant tous les deux dans ladite paroi latérale (20), un clapet anti-retour, tel une bille (4), disposée au niveau de l'extrémité interne (250) dudit conduit d'amenée (25), un ressort à lame (5), épousant la paroi latérale (20) de ladite chambre et poussant le clapet (4) vers son siège (250), un organe mobile (6) commandé depuis l'extérieur, muni de moyens de verrouillage dans une position déterminée, la longueur de la lame du ressort (5) agissant sur le clapet (4) étant déterminée par la position dudit organe mobile (6). Cette valve est remarquable en ce que ledit organe mobile (6) est constitué par un arceau (60) flexible, élastique, épousant la paroi intérieure cylindrique (20) de ladite chambre (21).

## Description

La présente invention concerne une valve sous-cutanée, destinée à la régulation du liquide céphalo-rachidien (LCR), dans le traitement de l'hydrocéphalie et son dispositif de réglage externe qui permet de modifier la pression d'ouverture de cette valve implantée, depuis l'extérieur, à travers les tissus cutanés.

Depuis plus de cinquante ans maintenant, on sait que l'hydrocéphalie peut être provoquée notamment par un dérèglement ou un blocage des sites naturels de résorption du liquide céphalo-rachidien (LCR), appelés villosités arachnoïdiennes.

Chez l'individu normal, le cerveau est maintenu à une pression hydrostatique constante, à l'intérieur de la cavité crânienne, grâce à un mécanisme régulateur d'ouverture et de fermeture de ces villosités arachnoïdiennes. Ce mécanisme permet normalement aux ventricules cérébraux qui contiennent le LCR de garder un gradient de pression constant. En conséquence, tout dérèglement de la résorption du LCR entraîne simultanément une augmentation du volume des ventricules cérébraux et dans la plupart des cas une augmentation de la pression intra-ventriculaire.

Chez les nouveau-nés dont les fontanelles sont encore ouvertes, l'hydrocéphalie se manifeste par une augmentation de volume de la boîte crânienne provoquée par l'augmentation de la pression intra-ventriculaire.

Chez l'adulte, en raison de la rigidité de la boite crânienne, l'hydrocéphalie se manifeste par des troubles de la démarche, des incontinences, des troubles mentaux et une dégradation progressive du parenchyme cérébral.

Un traitement de l'hydrocéphalie, couramment utilisé, consiste à dériver le liquide céphalo-rachidien contenu dans les ventricules de la cavité crânienne, vers un autre site de résorption tel que le coeur ou le péritoine.

A cet effet, on introduit un cathéter dans l'un des ventricules de la cavité crânienne en pratiquant un trou au trépan dans le crâne du patient, et on relie ledit cathéter à une autre tubulure qui est passée par voie sous-cutanée sous le cuir chevelu et qui évacue le LCR soit vers le coeur, soit vers le péritoine, dans la plupart des cas.

Lorsque les villosités arachnoïdiennes fonctionnent normalement, elles ont pour rôle de maintenir une pression constante entre le LCR et le sang veineux afin d'éviter que les ventricules cérébraux ne se vident en position orthostatique. Dans le cas où l'on installe un cathéter de dérivation pour effectuer la résorption du LCR, il est nécessaire d'incorporer sur le trajet de la dérivation, une valve de régulation de la pression intra-ventriculaire afin de rétablir à l'intérieur de la cavité crânienne, le gradient de pression défaillant.

On connaît déjà d'après les documents US-A-3 288 142 et US-A-3 527 226, une valve comportant un dispositif de contrôle de la pression d'un liquide constitué d'une bille formant clapet, disposée dans un siège tronconique et appliquée contre ce siège par un ressort calibré de façon à permettre l'ouverture de la valve à une pression déterminée. Toutefois, ce type de valve ne fonctionne que dans une gamme de pressions limitée, de sorte qu'il est nécessaire de fabriquer différentes valves, prévues pour fonctionner dans différentes gammes de pressions adaptées aux cas à traiter. D'autre part, il est parfois nécessaire de remplacer une première valve implantée initialement, si celle-ci vient à présenter une pression de fermeture trop élevée ou trop basse au cours de l'évolution de la maladie ou pendant son traitement.

Pour résoudre ces problèmes, on connaît d'après le document EP 0 060 369, une valve programmable dont la pression d'ouverture peut être réglée à tout moment par le neurochirurgien, depuis l'extérieur et de façon non invasive, en fonction des conditions physiologiques du patient en cours de traitement.

Cette valve programmable comprend une chambre cylindrique de faible épaisseur, un conduit d'amenée et un conduit d'évacuation du liquide céphalo-rachidien débouchant tous les deux dans la paroi cylindrique de ladite chambre, un clapet anti-retour (ou bille) placé au niveau de l'extrémité interne conique dudit conduit d'amenée et un ressort à lame poussant ladite bille vers son siège conique, ce ressort étant arqué de manière à épouser la paroi cylindrique de ladite chambre et l'une de ses extrémités étant fixée à l'extrémité d'un barreau magnétique rotatif (ou rotor) diamétral.

Le neurochirurgien peut commander depuis l'extérieur la rotation du barreau magnétique, à l'aide d'un aimant, ce qui a pour effet de faire varier le moment résistant du ressort au niveau du point de contact avec le clapet anti-retour. de la valve. En d'autres termes, la rotation du barreau magnétique a pour effet de faire varier la longueur de la partie active de la lame du ressort agissant sur la bille et donc de faire varier la pression d'ouverture de la valve.

L'immobilisation du rotor, et donc du ressort, est réalisée à l'aide d'une petite saillie prévue à l'extrémité du barreau, opposée à celle sur laquelle est fixé le ressort à lame, cette saillie pouvant se positionner dans différents évidements, ménagés dans la paroi cylindrique latérale de la chambre de la valve et correspondants à différentes pressions d'ouverture présélectionnées.

Une telle immobilisation du rotor s'est avérée suffisante dans la plupart des cas, toutefois, il est reconnu que certains champs magnétiques très puissants, tels que ceux utilisés lors d'une exploration par résonance magnétique nucléaire, pouvaient dérégler le rotor. On doit donc régler de nouveau celui-ci sur le patient, dès l'examen par RMN terminé.

Un autre inconvénient de la valve programmable décrite ci-dessus est la position diamétrale du barreau magnétique formant le rotor qui se trouve en travers du flux d'écoulement du liquide céphalo-rachidien (LCR).

Par ailleurs, on sait que la protéinémie du liquide céphalo-rachidien des patients atteints d'hydrocéphalie peut atteindre des taux allant jusqu'à 2 g %, entraînant une forte viscosité du LCR. En conséquence, dès qu'une valve présente au niveau de sa structure interne des points de retenue du LCR, elle aura tendance à s'obstruer facilement et rapidement.

Afin de résoudre le problème précité du dérèglement de la valve sous l'influence d'un champ magnétique très important, il a été proposé une valve sous-cutanée décrite dans le document FR 2 721 520.

Cette valve présente une structure proche de celle décrite ci-dessus en liaison avec le document EP 0 060 369, sauf que le rotor magnétique est constitué d'un barreau en forme de H dont les deux paires de branches latérales de part et d'autre de son axe de rotation central, servent de moyens de guidage à deux micro-aimants. Ces deux micro-aimants dont les faces en regard sont de mêmes polarités, peuvent coulisser entre les branches dudit rotor selon l'axe longitudinal du barreau, de manière à actionner des ergots de verrouillage. Ces ergots de verrouillage sont aptes à coopérer avec une série de cavités prévues sur la face latérale cylindrique de la chambre de la valve.

Cette valve résout le problème du dérèglement intempestif puisque si le patient est soumis à un fort champ magnétique unidirectionnel et que l'un des micro-aimants est attiré vers le centre de la valve en sortant ainsi de sa cavité de verrouillage, l'autre micro-aimant dont la face en regard est de même polarité sera repoussé encore davantage dans sa cavité de verrouillage. Les deux micro-aimants ne peuvent donc pas se désengager simultanément de leurs cavités de verrouillage respectives.

Toutefois, du fait même de la présence des deux pièces mobiles supplémentaires, le nombre de sites de retenue et donc d'espaces «morts» potentiels est encore accru et ceci d'autant plus que le mécanisme doit être extrêmement miniaturisé pour être intégré dans une chambre de valve dont les dimensions intérieures sont de l'ordre du centimètre. Une telle valve risque donc de s'obstruer encore plus rapidement que la valve du document EP 0 060 369.

La présente invention a donc pour but de résoudre les deux problèmes précités concernant l'immobilisation du ressort dans une position prédéterminée, même à l'intérieur d'un très fort champ magnétique et l'amélioration de la circulation du liquide céphalo-rachidien à l'intérieur de la chambre de la valve.

A cet effet, l'invention concerne une valve sous-cutanée pour le traitement de l'hydrocéphalie, ladite valve dont la pression d'ouverture est réglable depuis l'extérieur, comportant un corps de valve présentant une chambre interne à paroi latérale cylindrique, un conduit d'amenée et un conduit d'évacuation du liquide céphalo-rachidien débouchant tous les deux dans la paroi latérale de ladite chambre et aptes à être connectés respectivement à un cathéter d'amenée et à un cathéter de drainage dudit liquide, un clapet anti-retour, tel une bille, disposée au niveau de l'extrémité interne dudit conduit d'amenée, un ressort à lame courbe, épousant la paroi latérale de ladite chambre et poussant le clapet vers son siège, un organe magnétique mobile en rotation autour d'un axe **X-X'** et dont la rotation est commandée depuis l'extérieur de la valve et des moyens de verrouillage dudit organe mobile dans une position déterminée, la longueur de la partie active de la lame du ressort agissant sur le clapet étant déterminée par la position dudit organe mobile.

Conformément à l'invention, cet organe mobile est constitué par un arceau flexible, élastique, épousant la paroi intérieure cylindrique de la chambre sur au moins une partie de la circonférence de celle-ci tout en y exerçant une pression, cet arceau étant ainsi conformé qu'il ne contrarie pas le passage du liquide à travers ladite chambre, vers le conduit d'évacuation.

Ainsi, l'immobilisation de l'organe mobile est assurée par une répartition équilibrée des points de contact de l'arceau avec la paroi intérieure de la chambre, en supprimant tout contact en porte à faux qui pouvait résulter du seul point de fixation prévu à l'extrémité du barreau rotatif dans la valve du document EP 0 060 369.

Par ailleurs, la valve précitée offre une grande facilité de circulation du liquide à l'intérieur de la chambre puisque l'obstacle du barreau rotatif décrit dans les documents EP 0 060 369 et FR 2 721 520 est supprimé.

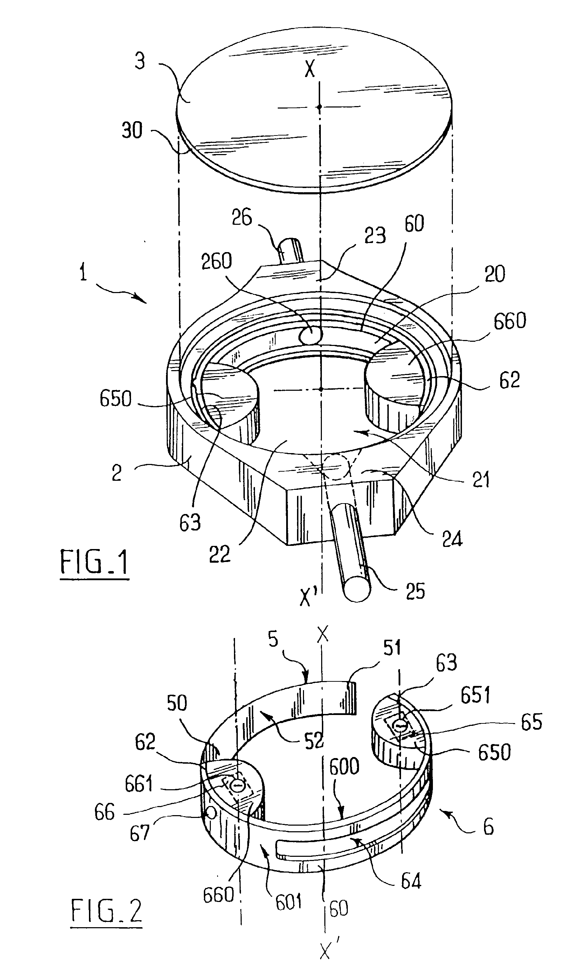

Selon d'autres caractéristiques avantageuses et non limitatives de l'invention :
- l'une des extrémités du ressort à lame est fixée sur la paroi cylindrique de la chambre, son autre extrémité étant libre, et l'une des extrémités de l'arceau peut coulisser sur la face intérieure (référencée 52 sur la figure 2) dudit ressort à lame, en prenant appui sur celle-ci afin d'y exercer une pression,
- l'une des extrémités du ressort à lame est fixée à l'une des extrémités de l'arceau, l'autre extrémité du ressort étant libre,
- l'arceau présente une ouverture pour le passage du liquide se trouvant dans la chambre interne, vers le conduit d'évacuation,
- le ressort à lame occupe au moins un tiers de la circonférence intérieure de ladite chambre,
- les moyens de verrouillage de l'organe mobile comprennent au moins deux bossages aptes à être reçus dans au moins deux cavités correspondantes, lesdits bossages étant disposés de façon diamétralement opposée sur la face extérieure de l'arceau et les cavités correspondantes étant ménagées dans la paroi latérale intérieure de la chambre ou inversement,

Selon un premier mode de réalisation, l'arceau porte sur sa face intérieure deux micro-aimants, fixés à chacune de ses deux extrémités de part et d'autre de l'axe de rotation vertical XX', et disposés verticalement de façon que leurs pôles respectifs de même signe soient dans un même plan et orientés vers le haut.

Selon un second mode de réalisation, l'arceau porte sur sa face intérieure deux micro-aimants, fixés à chacune de ses deux extrémités de part et d'autre de l'axe de rotation vertical XX', et disposés verticalement de façon que leurs pôles respectifs de signe contraire soient dans un même plan et orientés vers le haut.

L'invention concerne également un dispositif de réglage externe de la valve sous-cutanée précitée. La description suivante se réfère au premier mode de réalisation ci-dessus.

Ce dispositif comprend deux aimants disposés l'un par rapport à l'autre de_façon à ce qu'ils présentent chacun dans un même plan des pôles de même signe mais de telle sorte que ces pôles soient de signe contraire à ceux des deux micro-aimants de l'arceau, et enfin telle que la distance **L** entre leurs pôles soit légèrement inférieure à la distance **I** qui sépare les pôles des deux micro-aimants de l'arceau. Les deux aimants de ce dispositif ayant une masse magnétique très supérieure à celle des deux micro-aimants de l'arceau provoqueront à leur approche leur rétraction vers l'intérieur de la chambre autorisant ainsi la rotation dudit arceau.

Selon deux variantes de réalisation avantageuses de ce dispositif de réglage, celui-ci comprend :
- deux aimants enrobés dans un disque de résine, et disposés de telle sorte que leurs pôles respectifs de même signe dans un même plan et orientés vers le bas soient de signes opposés aux signes des pôles des micro-aimants de l'arceau ; ou
- deux aimants enrobés dans un disque de résine, et disposés de telle sorte que leurs pôles respectifs de signe contraire dans un même plan et orientés vers le bas soient de signes opposés aux signes des pôles des micro-aimants de l'arceau.

L'invention concerne également un dispositif de repérage externe de la position de l'arceau par l'utilisation d'une boussole ayant une aiguille non aimantée.

D'autres caractéristiques et avantages de l'invention apparaîtront à la lecture de la description suivante d'un mode de réalisation préféré de l'invention. Cette description est faite en référence aux dessins annexés dans lesquels :
- la figure 1 est une vue en perspective éclatée de la valve selon l'invention,
- la figure 2 est une vue en perspective de l'organe mobile de la valve,
- la figure 3 est une vue de dessus en coupe partielle de la valve selon l'invention,
- la figure 4 est une vue similaire à la figure 3, la valve étant réglée pour une pression d'ouverture différente, et
- la figure 5 est une vue en perspective de l'arceau de la valve et d'un mode de réalisation du dispositif de réglage selon l'invention.

La valve représentée en figure 1 comporte un corps de valve 1 constitué d'un boîtier cylindrique 2, fermé par un couvercle 3.

Cette valve présente un axe passant par le centre du couvercle 3 et perpendiculaire au plan de celui-ci, matérialisé par la ligne **X-X'**.

Ce boîtier 2 et ce couvercle 3 sont avantageusement réalisés dans un matériau bio-compatible, afin de rendre la valve implantable.

La valve sous-cutanée présente une chambre interne 21 cylindrique et de faible épaisseur, ménagée entre le fond 22 du boîtier, la paroi latérale cylindrique 20 de ce boîtier et le couvercle supérieur 3.

Le boîtier 2 présente deux prolongements 23, 24, diamétralement opposés, dans chacun desquels sont respectivement ménagés un conduit d'amenée 25 et un conduit d'évacuation 26 du LCR. Ces deux conduits débouchent dans la paroi latérale cylindrique 20 de la chambre, par des orifices respectifs 250, 260, diamétralement opposés.

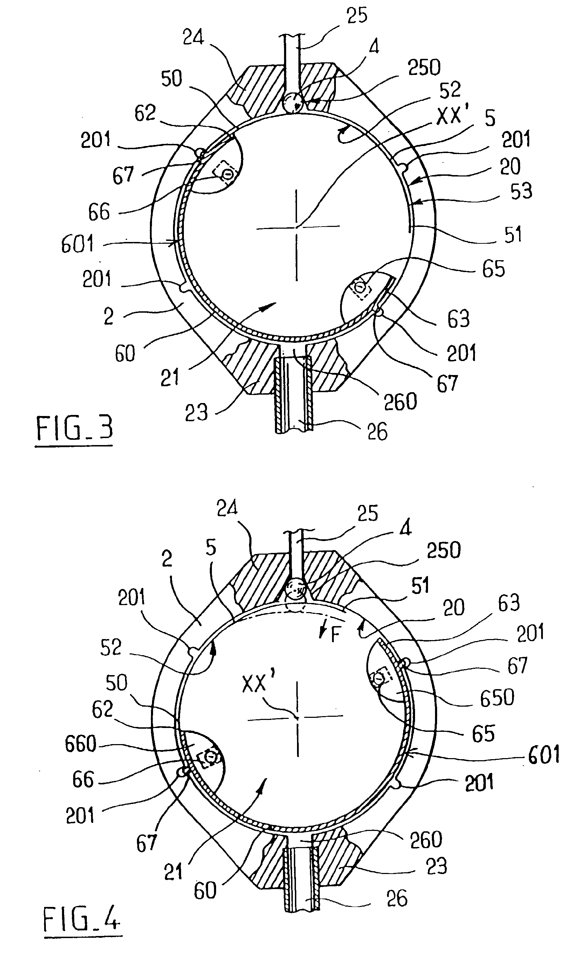

Comme cela apparaît mieux sur les figures 3 et 4, l'extrémité interne 250 du conduit d'amenée 25 présente une forme tronconique définissant un siège destiné à recevoir une bille 4 formant clapet anti-retour.

La bille 4 est maintenue contre son siège 250 par un ressort à lame 5, courbe, épousant la forme de la paroi latérale cylindrique 20 de la chambre 21. Ce ressort à lame 5 s'étend de façon avantageuse sur au moins un tiers de la circonférence intérieure de ladite chambre 21.

Comme cela apparaît mieux sur la figure 2, le ressort à lame 5 est fixé en porte à faux par l'une de ses extrémités 50, à un organe mobile 6 décrit ultérieurement, l'autre extrémité 51 de ce ressort à lame étant libre.

L'organe mobile 6 comprend un arceau 60 (ou lame arquée), flexible, élastique, réalisé en matière plastique ou en métal. Cet arceau 60 présente une face 600 dite intérieure, orientée vers l'intérieur de la chambre 21, une face extérieure 601 opposée et deux extrémités opposées, référencées respectivement 62 et 63, l'extrémité 62 correspondant à celle à laquelle l'extrémité 50 du ressort à lame 5 est fixée. Cet arceau 60 est disposé à l'intérieur de la chambre interne 21, de façon que sa face extérieure 601 épouse la forme de la paroi intérieure cylindrique 20 de ladite chambre et que cet arceau soit plaqué contre cette paroi 20 en y exerçant une certaine pression.

Cet arceau 60 s'étend devant l'extrémité interne 260 du conduit d'évacuation 26 et présente une ouverture 64 longitudinale, destinée à faciliter le passage du LCR depuis l'intérieur de la chambre 21, vers le conduit d'évacuation 26.

L'arceau 60 porte en outre sur sa face intérieure 600, deux micro-aimants 65, 66, disposés face à face. Chaque micro-aimant 65, respectivement 66, est noyé dans une masse de matière plastique biocompatible 650, respectivement 660, rendue solidaire de la face intérieure 600 de l'arceau 60.

Selon un premier mode de réalisation représenté sur la figure 2, les deux micro-aimants présentent dans un même plan leurs pôles respectifs 651 et 661 de même signe pointant vers le haut. Dans le cas de la figure 2, ces deux pôles sont négatifs, mais ils pourraient tout aussi bien être positifs.

Cette disposition particulière des aimants dans laquelle les pôles sont de même signe permet d'éviter l'effet de rotation intempestif qui est inévitable lorsque les deux pôles d'un même aimant sont de signes contraires, en particulier sous l'effet d'un champ magnétique très puissant comme celui utilisé dans un examen par RMN.

Grâce à un dispositif de réglage externe décrit ultérieurement, il est possible d'agir depuis l'extérieure de la valve, sur lesdits micro-aimants 65, 66, pour entraîner la rotation de l'arceau 60 autour de l'axe central **X-X'** de la chambre interne. Cet axe central **X-X'** constitue donc aussi un axe de rotation de l'organe mobile 6.

Enfin, comme on peut le voir sur les figures 2 à 4, l'arceau 60 présente des moyens de verrouillage dans une position prédéterminée. Ces moyens comprennent au moins deux bossages destinés à être reçus dans au moins deux cavités correspondantes.

Selon un exemple de réalisation illustré sur les figures 3 et 4, la face extérieure 601 de l'arceau 60 porte deux bossages 67 en saillie par rapport au plan de cette face et disposés de façon diamétralement opposée. La paroi latérale intérieure 20 de la chambre 21 présente quatre cavités 201 d'une forme correspondant sensiblement à celle des bossages 67.

Grâce à ces moyens de verrouillage, l'organe mobile 6 peut donc être immobilisé dans deux positions différentes correspondant à deux pressions différentes du ressort à lame 5 sur la bille 4.

Dans la position de l'organe mobile 6 illustrée en figure 4, le point de contact du ressort à lame 5 avec la bille 4 est éloigné du point de fixation du ressort 5 sur l'extrémité 62 de l'arceau 60, de sorte que le moment résistant du ressort s'opposant à la poussée exercée par le liquide sur la bille 4 est faible. Il en résulte que pour cette position de l'arceau la pression d'ouverture de la valve sera basse.

Au contraire, lorsque l'organe mobile 6 est dans la position illustrée en figure 3, le point de contact du ressort à lame 5 avec la bille 4 est proche du point de fixation du ressort 5 sur l'extrémité 62 de l'arceau 60, de sorte que le moment résistant du ressort est plus important. Il en résulte que pour cette position de l'arceau la pression d'ouverture de la valve sera haute.

Le réglage de la position de l'organe mobile 6 permet d'ajuster la pression d'ouverture de la valve sous-cutanée et donc la pression intra-ventriculaire du patient.

Sur les figures 3 et 4 et à des fins de simplification, seules deux fois deux cavités 201 ont été représentées pour deux positionnements différents de l'arceau. Il serait toutefois envisageable de prévoir un plus grand nombre de telles cavités pour augmenter le nombre de positions de réglage.

En outre, la nature élastique de l'arceau 60 permet à celui-ci de se comporter comme un ressort et de se plaquer contre la paroi cylindrique 20. On améliore ainsi l'immobilisation de l'organe mobile 6.

Par ailleurs, la circulation du liquide est grandement améliorée puisqu'il n'y a pas d'obstacle au milieu de la chambre 21, comme c'était le cas avec un rotor en forme de barreau.

Selon un second mode de réalisation non représenté sur les figures, le ressort à lame 5 est fixé par son extrémité 50 à la paroi cylindrique 20 de la chambre interne, tandis que son autre extrémité 51 est libre. Ce ressort 5 est en outre disposé comme représenté sur la figure 4, de façon que son extrémité libre 51 se trouve à faible distance du siège 250. Par ailleurs, l'arceau 60 est disposé de façon que son extrémité 62 et notamment le noyau de matière plastique 660 puisse coulisser sur la face intérieure 52 du ressort à lame 5, en prenant appui sur celle-ci afin d'y exercer une pression. C'est alors la position de cette extrémité 62 de l'arceau 60 qui détermine la valeur du moment résistant du ressort à lame 5.

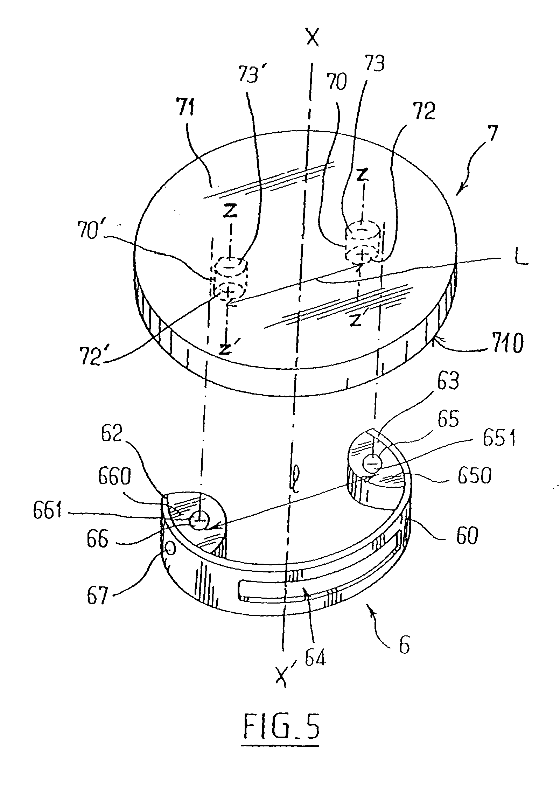

La rotation de l'arceau 60 est assurée par un dispositif de réglage externe dont un exemple de réalisation référencé 7 est illustré sur la figure 5. A des fins de simplification, sur cette figure, le ressort à lame 5 n'a pas été représenté.

Comme on peut le voir sur cette figure, le dispositif de réglage 7 comprend deux aimants cylindriques de forte puissance 70, 70', noyés par exemple dans un support tel qu'un disque de résine 71. Ces aimants sont disposés verticalement dans le disque c'est à dire perpendiculaires à son plan 710 mais de telle sorte qu'ils présentent leurs pôles de même signe dans le plan du disque. Ainsi qu'il apparaît dans l'exemple de la figure 5, ce sont leurs pôles positifs qui pointent vers le bas parce que les deux pôles des deux micro-aimants de l'arceau auxquels ils sont présentés sont des pôles négatifs.

En outre, l'écartement entre les deux aimants du dispositif de réglage a été calculé pour être d'une distance **L** entre leurs axes respectifs **Z, Z'** légèrement inférieure à celle **l** qui sépare les axes des deux micro-aimants de l'arceau.

Pour régler la pression d'ouverture de la valve, ce dispositif 7 est placé sur la tête du patient au-dessus du site de la valve de façon que les deux pôles positifs 72, 72' des aimants du dispositif de réglage soient au-dessus des deux micro-aimants de pôles négatifs de l'arceau. Selon les caractéristiques mentionnées ci-dessus, les deux pôles de l'aimant du dispositif de réglage provoqueront une rétraction des deux extrémités de l'arceau en l'écartant de la paroi intérieure 20, et toute rotation dudit dispositif permettra d'amener l'arceau dans la position désirée, et conjointement le ressort à lame 5 par rapport à la bille 4.

Dans le cas où les deux micro-aimants 65, 66 de l'arceau présentent leurs pôles positifs orientés vers le haut, les deux aimants 70 et 70' du dispositif de réglage, seront inversés de 180° de façon que leurs pôles négatifs respectifs 73, 73' soient au droit et en regard des deux pôles positifs des micro-aimants 65, 66.

Enfin, dans le dernier cas où les deux micro-aimants 65, 66 présentent leurs pôles respectifs de signe contraire pointés vers le haut, le dispositif de réglage 7 est identique à celui décrit conjointement avec la figure 5, si ce n'est que l'un de ses deux aimants est inversé de 180°.

Pour connaître la position de l'arceau dans la valve une fois que celle-ci est implantée, on utilisera une simple boussole dont la particularité est d'avoir une aiguille en fer doux non aimantée. Toute valve implantée est facilement repérable sous la peau du patient du fait de la petite protubérance qu'elle produit sur le cuir chevelu. En appliquant ladite boussole sur le site de la valve, l'aiguille indique immédiatement la position des deux micro-aimants de l'arceau, c'est à dire sa position.

## Revendications

1. Valve sous-cutanée pour le traitement de l'hydrocéphalie, ladite valve dont la pression d'ouverture est réglable depuis l'extérieur, comportant un corps de valve (1) présentant une chambre interne (21) à paroi latérale cylindrique (20), un conduit d'amenée (25) et un conduit d'évacuation (26) du liquide céphalo-rachidien débouchant tous les deux dans la paroi latérale (20) de ladite chambre (21) et aptes à être connectés respectivement à un cathéter d'amenée et à un cathéter de drainage dudit liquide, un clapet anti-retour, tel une bille (4), disposée au niveau de l'extrémité interne (250) dudit conduit d'amenée (25), un ressort à lame (5), courbe, épousant la paroi latérale (20) de ladite chambre et poussant le clapet (4) vers son siège (250), un organe magnétique (6) mobile en rotation autour d'un axe **X-X'** et dont la rotation est commandée depuis l'extérieur de la valve et des moyens de verrouillage dudit organe mobile (6) dans une position déterminée, la longueur de la partie active de la lame du ressort (5) agissant sur le clapet (4) étant déterminée par la position dudit organe mobile (6), **caractérisée en ce que** ledit organe mobile (6) est constitué par un arceau (60) flexible, élastique, épousant la paroi intérieure cylindrique (20) de ladite chambre (21), sur au moins une partie de la circonférence de celle-ci, tout en y exerçant une pression, cet arceau (60) étant ainsi conformé qu'il ne contrarie pas le passage du liquide à travers ladite chambre (21), vers le conduit d'évacuation (26).

2. Valve sous-cutanée selon la revendication 1, **caractérisée en ce que** l'une des extrémités (50) du ressort à lame (5) est fixée sur la paroi cylindrique (20) de ladite chambre, son autre extrémité (51) étant libre, et **en ce que** l'une des extrémités (62, 660) de l'arceau (60) peut coulisser sur la face intérieure (52) dudit ressort à lame, en prenant appui sur celle-ci afin d'y exercer une pression.

3. Valve sous-cutanée selon la revendication 1, **caractérisée en ce que** l'une des extrémités (50) du ressort à lame (5) est fixée à l'une des extrémités (62) de l'arceau (60), l'autre extrémité (51) du ressort étant libre.

4. Valve sous-cutanée selon l'une quelconque des revendications précédentes, **caractérisée en ce que** l'arceau (60) présente une ouverture (64) pour le passage du liquide se trouvant dans la chambre interne (21), vers le conduit d'évacuation (26).

5. Valve sous-cutanée selon l'une quelconque des revendications précédentes, **caractérisée en ce que** le ressort à lame (5) occupe au moins un tiers de la circonférence intérieure de ladite chambre (21).

6. Valve sous-cutanée selon l'une quelconque des revendications précédentes, **caractérisée en ce que** les moyens de verrouillage de l'organe mobile (6) comprennent au moins deux bossages (67) aptes à être reçus dans au moins deux cavités (201) correspondantes, lesdits bossages (67) étant disposés de façon diamétralement opposée sur la face extérieure (601) de l'arceau (60) et les cavités correspondantes (201) étant ménagées dans la paroi latérale intérieure (20) de ladite chambre (21) ou inversement.

7. Valve sous-cutanée selon l'une quelconque des revendications 1 à 6, **caractérisée en ce que** l'arceau (60) porte sur sa face intérieure (600) deux micro-aimants (65, 66), fixés à chacune de ses extrémités de part et d'autre de l'axe de rotation vertical **X-X'** et disposés verticalement, de façon que leurs pôles respectifs de même signe soient dans un même plan et orientés vers le haut.

8. Valve sous-cutanée selon l'une quelconque des revendications 1 à 6, **caractérisée en ce que** l'arceau (60) porte sur sa face intérieure (600) deux micro-aimants (65, 66), fixés à chacune de ses deux extrémités, de part et d'autre de l'axe de rotation vertical **X-X'** et disposés verticalement, de façon que leurs pôles respectifs de signe contraire soient dans un même plan et orientés vers le haut.

9. Dispositif magnétique de réglage externe de la valve sous-cutanée selon la revendication 7, **caractérisé en ce qu'**il comprend deux aimants (70, 70') enrobés dans un disque de résine (71), et disposés de telle sorte que leurs pôles respectifs de même signe dans un même plan et orientés vers le bas soient de signes opposés aux signes des pôles des micro-aimants de l'arceau.

10. Dispositif magnétique de réglage externe de la valve sous-cutanée selon la revendication 8, **caractérisé en ce qu'**il comprend deux aimants (70, 70') enrobés dans un disque de résine (71), et disposés de telle sorte que leurs pôles respectifs de signe contraire dans un même plan et orientés vers le bas soient de signes opposés aux signes des pôles des micro-aimants de l'arceau.

11. Dispositif de repérage externe de la position de l'arceau par l'utilisation d'une boussole ayant une aiguille non aimantée.
